# EUROPEAN PATENT APPLICATION

(11) **EP 2 905 023 A1**
(43) Date of publication of application: **12.08.2015**
(21) Application number: 15150331.5
(22) Date of filing: 11.06.2009
(51) Int. Cl.: A61K 31/497, A61K 31/506, A61K 45/06, A61L 31/16, A61P 41/00

(54) **Use of histamine H4 antagonist for the treatment of post-operative adhesions**

(30) Priority: 12.06.2008 US 61046 P
(62) Divisional of application: 09763577.5
(71) Applicant: Janssen Pharmaceutica N.V., 2340 Beerse (BE)
(72) Inventor: Thurmond, Robin, L., San Diego CA, 92107 (US); Wadsworth, Scott, A., New Hope PA, 18938 (US)
(74) Representative: Carpmaels & Ransford LLP

(57) **Abstract**

The present invention includes methods for the inhibition of post-operative adhesion formation between tissue surfaces in a body cavity having been subjected to a surgical procedure, which methods involve administering a histamine H4 receptor antagonist, systemically, directly to tissue surfaces in the body cavity, or both, and to delivery vehicles and compositions suitable for use for local, non-systemic administration of a drug to the body and directly to tissue within a body cavity having been subjected to a surgical procedure. The drug is selected from the group consisting of benzoimidazol-2-yl pyrimidines and pyrazines.

## Description

### FIELD OF THE INVENTION

The present invention relates to the use of a histamine H4 receptor antagonist to inhibit or prevent post-operative adhesion formation between tissue surfaces in a body cavity and to compositions containing a histamine H4 receptor antagonist for administration to the body for inhibition or prevention of post-operative adhesions.

### BACKGROUND OF THE INVENTION

Adhesion formation, in particular following peritoneal, thoracic, and spinal surgery, for example, is a major source of postoperative morbidity and mortality. Appendectomy and gynecologic surgery, for example, are the most frequent surgical procedures implicated in clinically significant adhesion formation. The most serious complication of intraperitoneal adhesions is intestinal obstruction. In addition, adhesions are associated with chronic or recurrent pelvic pain and infertility in females, nerve compression and pain in the spine, post-operative complications following thoracic surgery, and loss of mobility in the hand after reconstructive surgery.

The pathogenesis of adhesion formation is complex and not entirely understood. The first step is believed to involve excess fibrin deposition to form a scaffold. Organization of the fibrin scaffold by cellular elements, including cells such as fibroblasts, then follows.

Various approaches for the prevention of adhesion formation have been actively explored (diZerega, G. S. & Rodgers, K. E., "Prevention of Postoperative Adhesions," in "The Peritoneum," diZerega, G. S. & Rodgers, K. E., eds., Springer-Verlag, New York, pp. 307- 369 (1992)). In general, the treatments fall into one of several categories: limiting tissue apposition; reduction of local tissue inflammation; prevention of fibrin deposition and removal of fibrin deposits; reduction of the proliferation of cells such as fibroblasts; and collagen inhibition.

For example, physical barriers have been used in attempts to prevent adhesion formation by limiting tissue apposition during the critical period of healing, thereby minimizing the development of fibrin matrix between tissue surfaces. Barrier agents that have been employed include both mechanical barriers and viscous solutions. Mixed efficacy results have been obtained using film barriers such as poly(tetrafluoroethylene). Such a membrane also is less than ideal, as it must be sutured into place and is nonabsorbable. Absorbable barriers would be preferable, but some studies have demonstrated the efficacy of such barriers to be less than ideal in preventing adhesions. Liquid barriers also have been considered for use in preventing adhesions; for example, both chondroitin sulfate and carboxymethyl cellulose have shown some promise in animal models.

Another approach that has been explored involves the removal of fibrin deposits. Although proteolytic enzymes (e.g., pepsin, trypsin and papain) should theoretically augment the local fibrinolytic system and limit adhesion formation, these enzymes are neutralized rapidly by peritoneal exudates, rendering them virtually useless for adhesion prophylaxis. While various fibrinolytics, for example, fibrinolysin, streptokinase and urokinase, have been advocated, a potential complication to the clinical use of these enzymes in postoperative therapy is excessive bleeding resulting from their administration.

Additionally, collagen inhibitors have been evaluated. The biosynthesis of collagen involves unique post-translational modification of pro-alpha chains. Hydroxylation of prolyl and lysyl residues, a key step in collagen formation, is vital for normal triple-helix formation and intermolecular cross-linking. When post-translational processing is inhibited, non-helical procollagen forms, which then is degraded by intracellular proteases and secreted into the extracellular matrix at a slow rate as a nonfunctional protein. The incorporation of proline analogs, e.g., cis-4-hydroxy-L-proline (cHyp) into nascent pro-alpha chains has been shown to reduce the extracellular accumulation of collagen. Such agents are believed to act more generally by inhibiting collagen synthesis and thereby averting certain of the pathophysiological sequelae of fibrosis, such as atherosclerosis and hypertension. Through the distortion of bond angles and from steric hindrance among polypeptide chains, cHyp inhibits the folding of pro-alpha chains into a stable triple helix. Other proline analogs, such as cis-4-fluoroproline, cis-4- bromoproline, and 3,4-dehydroproline, have similar effects, but also can inhibit other post-translational steps. The compound 3,4-dehydroproline is an example of a proline analog that also can inhibit other post-translational steps. For example, 3,4-dehydroproline inhibits prolyl hydroxylase activity. Unfortunately, it also is recognized that cHyp can be potentially toxic if used improperly, particularly in chronic use, and thus has had limited clinical utility.

The compound N-(3,4-dimethoxycinnamoyl) anthranilic acid, commonly known as Tranilast, also has been examined as an adhesion prevention agent in rats, (Shinya, A., et.al. (1999), "The Prevention of Postoperative Intraperitoneal Adhesions by Tranilast: N-(3,4-dimethoxycinnamoyl) Anthranilic Acid," Jpn J Surg. 29:51-54). In this study, Shinya, et al. used oral, systemic dosing both pre- and post-operatively in a rat intraperitoneal adhesion model. Of significant note, however, ischemia via abrasion of the surgical site was not performed in the model utilized in this study. Therefore, it is believed that the type of trauma necessary to cause loss of blood flow and to more accurately mimic the clinical situation in humans was not present. It is such a loss of blood flow that strongly contributes to reduced tissue plasminogen activity, fibrin deposition and adhesion formation. Accordingly, it is believed that the value and validity of such a study with respect to the efficacy of systematic administration of Tranilast for inhibition or prevention of adhesions is questionable.

Anti-inflammatory drugs have been evaluated for their effects on postoperative adhesion formation, as they may limit the release of fibrinous exudate in response to inflammation at the surgical site. Two general classes of these drugs have been tested: corticosteroids and nonsteroidal anti-inflammatory drugs. The results of corticosteroid use in animal studies generally have not been encouraging, and clinical use of corticosteroids is limited by their other pharmacological properties. Nonsteroidal anti-inflammatory drugs show promise for inhibition of postoperative adhesion formation (Rodgers, K. E., "Nonsteroidal anti-inflammatory drugs (NSAIDs) in the treatment of Postsurgical adhesion," in "Treatment of Post-Surgical Adhesions," diZerega, G. S. et al., eds., Wiley-Liss, New York, pp. 119-129 (1990)). However, as described herein, a different class of anti-inflammatory compounds, the histamine H4 receptor antagonists, has demonstrated effective inhibition and prevention of post-operative adhesions in *in vivo* models.

Modulation of H4 receptors controls the release of inflammatory mediators and inhibits leukocyte recruitment, thus providing the ability to prevent and/or treat H4-mediated diseases and conditions, including the deleterious effects of inflammation. Because of its preferential expression on immunocompetent cells, the H₄ receptor is closely related with the regulatory functions of histamine during the immune response.

A biological activity of histamine in the context of immunology and autoimmune diseases is closely related with the allergic response and its deleterious effects, such as inflammation. Events that elicit the inflammatory response include physical stimulation (including trauma), chemical stimulation, infection, and invasion by a foreign body. The inflammatory response is characterized by pain, increased temperature, redness, swelling, reduced function, or a combination of these.

Mast cell degranulation (exocytosis) releases histamine and leads to an inflammatory response that may be initially characterized by a histamine-modulated wheal and flare reaction. A wide variety of immunological stimuli (e.g., allergens or antibodies) and non-immunological (e.g., chemical) stimuli may cause the activation, recruitment, and de-granulation of mast cells. Mast cell activation initiates allergic inflammatory responses, which in turn cause the recruitment of other effector cells that further contribute to the inflammatory response. It has been shown that histamine induces chemotaxis of mouse mast cells (Hofstra, et al., 2003). Chemotaxis does not occur using mast cells derived from H₄ receptor knockout mice. Furthermore, the response is blocked by an H₄-specific antagonist, but not by H₁, H₂ or H₃ receptor antagonists (Hofstra, et al., 2003; Thurmond, R.L., et al., J. Pharmacol. Exp. Ther. 2004, 309(1), 404-413). The chemotactic response of mast cells to histamine is mediated by histamine H₄ receptors. (Thurmond, et al., 2004).

It has been shown that eosinophils can chemotax towards histamine (O'Reilly, M., et al., J. Recept. Signal Transduction 2002, 22(1-4), 431-448; Buckland, K.F., et al., Br. J. Pharmacol. 2003, 140(6), 1117-1127; Ling et al., 2004). Using H₄ selective ligands, it has been shown that histamine-induced chemotaxis of eosinophils is mediated through the H₄ receptor (Buckland, et al., 2003; Ling et al., 2004). Cell surface expression of adhesion molecules CD11b/CD18 (LFA-1) and CD54 (ICAM-1) on eosinophils increases after histamine treatment (Ling, et al., 2004). This increase is blocked by H₄ receptor antagonists but not by H₁, H₂, or H₃ receptor antagonists.

The histamine H4 receptor (H₄R) also plays a role in dendritic cells and T cells. In human monocyte-derived dendritic cells, H₄R stimulation suppresses IL-12p70 production and drives histamine-mediated chemotaxis (Gutzmer, R., et al., J. Immunol. 2005, 174(9), 5224-5232). A role for the H₄ receptor in CD8⁺ T cells has also been reported. Gantner, et al., (2002) showed that both H₄ and H₂ receptors control histamine-induced IL-16 release from human CD8⁺ T cells. IL-16 is found in the bronchoalveolar fluid of allergen- or histamine-challenged asthmatics (Mashikian, V.M., et al., J. Allergy Clin. Immunol. 1998, 101 (6, Part 1), 786-792; Krug, N., et al., Am. J. Resp. Crit. Care Med. 2000, 162(1), 105-111) and is considered important in CD4⁺ cell migration. The activity of the receptor in these cell types indicates an important role in adaptive immune responses such as those active in autoimmune diseases.

In vivo H₄ receptor antagonists were able to block neutrophillia in zymosan-induced peritonitis or pleurisy models (Takeshita, K., et al., J. Pharmacol. Exp. Ther. 2003, 307(3), 1072-1078; Thurmond, et al., 2004). In addition, H₄ receptor antagonists have activity in a widely used and well-characterized model of colitis (Varga, C., et al., Eur. J. Pharmacol. 2005, 522(1-3), 130-138). These results support the conclusion that H₄ receptor antagonists have the capacity to be anti-inflammatory in vivo.

The present invention is directed to the use of a histamine H4 receptor antagonist to inhibit or prevent the formation of post-operative adhesions. Such compounds, and compositions thereof, may be delivered directly to the surgical site or systemically to inhibit or prevent the formation of post-operative adhesions.

### SUMMARY OF THE INVENTION

The present invention is directed to methods for inhibiting post-operative adhesion formation in a body between tissue surfaces in a body cavity having been subjected to a surgical procedure, comprising administering an effective amount of at least one histamine H4 receptor antagonist to the tissue surfaces in the body cavity. In some embodiments, compounds are administered non-systemically (e.g. directly). In other embodiments, compounds are administered by systemic administration, either before, during, or after surgery, or at a combination of times. In still other embodiments, compounds are administered by both non-systemic and systemic administration.

In another general aspect, the present invention is directed to a delivery vehicle suitable for local, non-systemic administration of a drug directly to tissue within a body cavity having been subjected to a surgical procedure, the vehicle comprising an effective amount of at least one histamine H4 receptor antagonist.

In another general aspect, the present invention is directed to pharmaceutical compositions suitable for use in local, non-systemic administration of a drug directly to tissue within a body cavity having been subjected to a surgical procedure, where the composition comprises an effective amount of at least one histamine H4 receptor antagonist. The pharmaceutical composition further comprises a carrier suitable for local, non-systemic administration of the at least on histamine H4 receptor antagonist.

An object of the present invention is to overcome or ameliorate at least one of the disadvantages of the conventional methodologies and/or prior art, or to provide a useful alternative thereto.

Additional embodiments, features, and advantages of the invention will be apparent from the following detailed description and through practice of the invention.

### DETAILED DESCRIPTION OF THE INVENTION

According to the present invention, methods for the administration of certain histamine H4 receptor antagonists and pharmaceutical compositions suitable for use in systemic or non-systemic administration of such compounds to the body tissue are useful in inhibiting or preventing formation of adhesions between tissue and/or organ surfaces, the most common cause of which is prior surgery. While prevention of the formation of any adhesions after surgery would be preferred, it is sufficient to inhibit formation of such adhesions such that the degree or extent of adhesion formation is low enough not to present serious problems associated with adhesion formation, such as are described herein.

The inventive methods and compositions have been shown to be especially effective in inhibiting adhesion formation in the peritoneum following surgery. The present invention also finds utility in other contexts, e.g., for cardiovascular, orthopedic, thoracic, ophthalmic, CNS, reconstructive surgery, e.g. hand, and other uses, where the formation of adhesions is a significant concern. In addition, inhibition of adhesion formation or drug loculation during the intraperitoneal administration of a chemotherapeutic agent, or inhibition of adhesion formation or drug loculation during the administration of a pain medication such as morphine also would be desirable. As such, the combination of a histamine H4 receptor antagonist with pharmaceutical compositions containing the chemotherapeutic agent or other therapeutic agents in order to provide not only the therapeutic affect sought by the therapeutic agents, but also to inhibit the formation of adhesions that may form as a result of administration of such pharmaceutical compositions, are encompassed by the scope of the present invention.

The present invention is based on the discovery that H4 receptor antagonist compounds, such as (5-chloro-1H-indol-3-yl)-(4-methyl-piperazin-1-yl)-methanone and [5-(4,6-dimethyl-1H-benzoimidazol-2-yl)-4-methyl-pyrimidin-2-yl]-[3-(1-methyl-piperidin-4-yl)-propyl]-amine, compounds known for their activity in the H4 receptor that is involved in conditions such as inflammation, allergies and asthma, are useful in reducing or preventing formation of adhesions between tissue surfaces in body cavities following surgical procedures when administered directly to the tissue and body cavity in amounts and under conditions effective to inhibit the formation of post-operative adhesions. In addition, these compounds are useful when administered systemically before and/or after surgery.

The processes that are involved in adhesion formation include, but are not limited to inflammatory responses, cell growth and differentiation, angiogenesis, extracellular matrix turnover, tissue remodeling, and apoptosis (Chegini, N (2002), "Peritoneal Molecular Environment, Adhesion Formation and Clinical Implication," Frontiers in Bioscience 7, e91-115, April 1, 2002). However, no one of these possible mechanisms of action in and of itself would be likely to be sufficient to enable one to predict whether histamine H4 receptor antagonist compounds would have any utility in the reduction of adhesion formation.

Though specific embodiments disclosed herein exemplify (5-chloro-1H-indol-3-yl)-(4-methyl-piperazin-1-yl)-methanone and [5-(4,6-dimethyl-1H-benzoimidazol-2-yl)-4-methyl-pyrimidin-2-yl]-[3-(1-methyl-piperidin-4-yl)-propyl]-amine as useful compounds for inhibiting or preventing post-surgical adhesion formation, it is understood that other histamine H4 receptor antagonists are contemplated as being suitable for use in the present invention. Suitable histamine H4 receptor antagonists contemplated for their use in the methods and pharmaceutical compositions of the invention include:
a) benzoimidazolyl and benzoxazolyl amides and thioamides, as described in US 2003/0207893 (Nov. 16, 2003) ;
b) 8-membered bicyclic aromatic amides and thioamides, as described in US 2004/0048878 (March 11, 2004);
c) 9-membered bicyclic aromatic amides, thioamides, and imides, as described in US 2004/0058934 (March 25, 2004);
b) amino-substituted quinoxalines, as described in US 2005/0070527 (March 31, 2005);
d) 2-arylbenzimidazole ether compounds, as described in US 2005/0070550 (March 31, 2005);
e) 2-arylimidazole ether compounds, as described in US 2005/0261309 (Nov. 24, 2005);
f) benzoimidazol-2-yl pyridines, as described in US 2007/0232616 (Oct. 4, 2007);
g) indolyl and benzimidazolyl pyrrolidinyl amides, as described in US 2007/0238771 (Oct. 11, 2007);
h) benzoimidazol-2-yl pyrimidines and pyrazines, as described in US 2007/0244126 (Oct. 18, 2007);
i) benzofuro- and benzothienopyrimidines, as described in US 2008/0015200 (Jan. 17, 2008);
j) 2-aminopyrimidines, as described in U.S. Pat. Appl. 12/070,051 (Feb. 14, 2008);
k) aryl-substitued pyridines and triazines, as described in U.S. Provisional Pat. Appl. 60/971,676 (Sept. 12, 2007);
l) thieno- and furo-pyrimidines, as described in U.S. Provisional Pat. Appl. 60/972,589 (Sept. 14, 2007); and
m) bicyclic heteroaryl-substituted imidazoles, as described in 61/014,572 (Dec. 18, 2007);
   pharmaceutically acceptable salts, hydrates, solvates and mixtures of such compounds.

All the publications listed in a)-m) above are incorporated herein by reference in their entirety.

In some embodiments, suitable histamine H4 receptor antagonists are any one of benzoimidazolyl and benzoxazolyl amides and thioamides, as described in US 2003/0207893 (Nov. 16, 2003), benzoimidazol-2-yl pyrimidines and pyrazines, as described in US 2007/0244126 (Oct. 18, 2007), pharmaceutically acceptable salts, hydrates, solvates, and mixtures thereof. In other embodiments, suitable histamine H4 receptor antagonists are any one of (5-chloro-1H-indol-3-yl)-(4-methyl-piperazin-1-yl)-methanone, [5-(4,6-dimethyl-1H-benzoimidazol-2-yl)-4-methyl-pyrimidin-2-yl]-[3-(1-methyl-piperidin-4-yl)-propyl]-amine, pharmaceutically acceptable salts, hydrates, solvates and mixtures thereof.

A "pharmaceutically acceptable salt" is intended to mean a salt of a free acid or base of a histamine H4 receptor antagonist that is non-toxic, biologically tolerable, or otherwise biologically suitable for administration to the subject. See, generally, S.M. Berge, et al., "Pharmaceutical Salts", J. Pharm. Sci., 1977, 66:1-19, and Handbook of Pharmaceutical Salts, Properties, Selection, and Use, Stahl and Wermuth, Eds., Wiley-VCH and VHCA, Zurich, 2002. Preferred pharmaceutically acceptable salts are those that are pharmacologically effective and suitable for contact with the tissues of patients without undue toxicity, irritation, or allergic response. A histamine H4 receptor antagonist may possess a sufficiently acidic group, a sufficiently basic group, or both types of functional groups, and accordingly react with a number of inorganic or organic bases, and inorganic and organic acids, to form a pharmaceutically acceptable salt. Examples of pharmaceutically acceptable salts include sulfates, pyrosulfates, bisulfates, sulfites, bisulfites, phosphates, monohydrogen-phosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, γ-hydroxybutyrates, glycolates, tartrates, methane-sulfonates, propanesulfonates, naphthalene-1-sulfonates, naphthalene-2-sulfonates, and mandelates.

If a histamine H4 receptor antagonist contains a basic nitrogen, the desired pharmaceutically acceptable salt may be prepared by any suitable method available in the art, for example, treatment of the free base with an inorganic acid, such as hydrochloric acid, hydrobromic acid, sulfuric acid, sulfamic acid, nitric acid, boric acid, phosphoric acid, and the like, or with an organic acid, such as acetic acid, phenylacetic acid, propionic acid, stearic acid, lactic acid, ascorbic acid, maleic acid, hydroxymaleic acid, isethionic acid, succinic acid, valeric acid, fumaric acid, malonic acid, pyruvic acid, oxalic acid, glycolic acid, salicylic acid, oleic acid, palmitic acid, lauric acid, a pyranosidyl acid, such as glucuronic acid or galacturonic acid, an alpha-hydroxy acid, such as mandelic acid, citric acid, or tartaric acid, an amino acid, such as aspartic acid or glutamic acid, an aromatic acid, such as benzoic acid, 2-acetoxybenzoic acid, naphthoic acid, or cinnamic acid, a sulfonic acid, such as laurylsulfonic acid, p-toluenesulfonic acid, methanesulfonic acid, ethanesulfonic acid, any compatible mixture of acids such as those given as examples herein, and any other acid and mixture thereof that are regarded as equivalents or acceptable substitutes in light of the ordinary level of skill in this technology.

If the histamine H4 receptor antagonist is an acid, such as a carboxylic acid or sulfonic acid, the desired pharmaceutically acceptable salt may be prepared by any suitable method, for example, treatment of the free acid with an inorganic or organic base, such as an amine (primary, secondary or tertiary), an alkali metal hydroxide, alkaline earth metal hydroxide, any compatible mixture of bases such as those given as examples herein, and any other base and mixture thereof that are regarded as equivalents or acceptable substitutes in light of the ordinary level of skill in this technology. Illustrative examples of suitable salts include organic salts derived from amino acids, such as glycine and arginine, ammonia, carbonates, bicarbonates, primary, secondary, and tertiary amines, and cyclic amines, such as benzylamines, pyrrolidines, piperidine, morpholine, and piperazine, and inorganic salts derived from sodium, calcium, potassium, magnesium, manganese, iron, copper, zinc, aluminum, and lithium.

Preferred histamine H4 receptor antagonists, including their pharmaceutically acceptable salts, hydrates, solvates and mixtures thereof, are those that exhibit little or no toxicity both at the local and systemic level and are suitable for use in animals, including humans. One skilled in the art will be able to readily identify those analogs once having the benefit of this disclosure.

In methods of the invention, histamine H4 receptor antagonists are used to inhibit the formation of post-operative adhesions. The term "inhibit" or "inhibiting" as used herein is intended to refer to administration of an active agent or composition of the invention to a body cavity for the purpose of effecting a therapeutic or prophylactic benefit through modulation of histamine H₄ receptor activity. Inhibiting includes reversing, ameliorating, alleviating, inhibiting the progress of, lessening the severity of, or preventing a post-operative adhesion.

Pursuant to the present invention, suitable histamine H4 receptor antagonists are administered and maintained at an effective concentration at the site of potential adhesion formation for a period of time sufficient to prevent adhesion formation. Histamine H4 receptor antagonists typically are administered systemically or non-systemically (e.g., directly to the body cavity), or both, over a post-operative interval until healing of the wound site is complete. In some embodiments, histamine H4 receptor antagonists may be delivered in a single dose and maintained in contact with the tissue in the body cavity as described herein. In other embodiments, histamine H4 receptor antagonists may be delivered in a series of doses timed to continue the administration over a period of time sufficient to inhibit adhesion formation, i.e. by sustained release.

An "effective amount" is an amount effective to inhibit formation of post-operative adhesions upon administration of the histamine H4 receptor antagonist to the tissue under conditions effective to provide inhibition of post-operative adhesions in the body cavity. The therapeutically effective concentrations of a histamine H4 receptor antagonist are ones that inhibit or prevent post-surgical adhesion formation between tissue surfaces in body cavities having undergone surgery when applied to tissue in the body cavity. The minimum amount of a histamine H4 receptor antagonist that can be administered must be effective to inhibit formation of the post-operative adhesion, as described herein. The maximum amount of a histamine H4 receptor antagonist that may be administered is limited by the toxicity of the compound. Effective amounts or doses of the active agents of the present invention may be ascertained by routine methods such as modeling, dose escalation studies or clinical trials, and by taking into consideration routine factors, e.g., the mode or route of administration or drug delivery, the pharmacokinetics of the agent, the severity and course of the disease, disorder, or condition, the subject's previous or ongoing therapy, the subject's health status and response to drugs, and the judgment of the treating physician. An exemplary dose is in the range of from about 0.001 to about 200 mg of histamine H4 receptor antagonist per kg of subject's body weight per day, preferably about 0.05 to 100 mg/kg/day, or about 1 to 35 mg/kg/day, or about 0.1 to 10 mg/kg daily in single or divided dosage units (e.g., BID, TID, QID). For a 70-kg human, an illustrative range for a suitable dosage amount is from about 1 to 200 mg/day, or about 5 to 50 mg/day.

Once improvement of the post-operative adhesion has occurred, the dose may be adjusted for preventive or maintenance treatment. For example, the dosage or the frequency of administration, or both, may be reduced as a function of the symptoms, to a level at which the desired therapeutic or prophylactic effect is maintained. Of course, if symptoms have been alleviated to an appropriate level, treatment may cease. Patients may, however, require intermittent treatment on a long-term basis upon any recurrence of symptoms.

The present invention is directed to pharmaceutical compositions comprising a histamine H4 receptor antagonist as described above. In some embodiments, the pharmaceutical composition additionally comprises a pharmaceutically acceptable excipient.

In some embodiments, the histamine H4 receptor antagonist is administered non-systemically, e.g., directly to the body cavity on which the surgery was performed. In other embodiments, the histamine H4 receptor antagonist is administered systemically by a suitable route of administration, e.g., oral, parenteral, rectal, topical, or ocular routes, or by inhalation. In addition, the histamine H4 receptor antagonist may be administered systemically in conjunction with local, non-systemic administration of the histamine H4 receptor antagonist.

In one embodiment of the invention, a histamine H4 receptor antagonist is administered directly to a targeted injury site, following the surgical procedure conducted at the site, by a delivery vehicle suitable for local, non-systemic administration of a therapeutic agent to the tissue surfaces, for example, a poly(ethylene glycol)/sodium carboxymethylcellulose aqueous gel, in order to reduce, or inhibit, or prevent adhesion formation at the site after surgery. Preferably, at least one histamine H4 receptor antagonist is administered in a single dose prior to skin closure after surgery using a delivery vehicle that enables the maintenance of requisite effective concentrations of the compound for a period of time sufficient to prevent adhesion formation during healing of the site. A suitable delivery vehicle itself essentially would be non-inflammatory and non-immunogenic and would permit release of the histamine H4 receptor antagonist so as to maintain effective levels thereof over the desired period of time.

A large variety of alternative sustained release delivery vehicles for administering the histamine H4 receptor antagonist also are contemplated as within the scope of the present invention when containing therapeutically effective amounts of the histamine H4 receptor antagonist. Suitable delivery vehicles include, but are not limited to, at least one of nanoparticles, microcapsules or microspheres; liposomes and other lipid-based release systems; absorbable and/or biodegradable mechanical barriers; polymeric delivery materials such as, but not limited to, at least one of polyethylene oxide/polypropylene oxide block copolymers (i.e., poloxamers), poly(orthoester)s, poly(vinyl alcohol)s, poly(anhydride)s, poly(methacrylate)s, poly(methacryladmide)s, anionic carbohydrate polymers, poly(hydroxybutyric acid)s, and polyacetals. Most preferably, a suitable formulation to achieve the most desired release profile of the histamine H4 receptor antagonist, a near pseudo zero-order, comprises injectable microcapsules or microspheres prepared from a biodegradable polymer such as, but not limited to, at least one of poly(1-lactide), poly(dl-lactide), poly(dl-lactide-co-glycolide)s, poly(1-lactide-co-glycolide)s, poly(e-caprolactone), polyglycolide, poly(p-dioxanone)s, poly(trimethylene carbonate), poly(alkylene diglycolate)s, poly(oxaester)s, poly(oxaamide)s, , and copolymers and blends thereof. Other desired release profiles, such as ones that yield an initial burst release of the histamine H4 receptor antagonist followed by zero-order sustained release (burst/sustained release), may be created by mixing encapsulated and non-encapsulated drug into the formulation.

Glycerides, useful long chain carboxylic acid esters include, but are not limited to, at least one of those from the group of: glyceryl monostearates; glyceryl monopalmitates; mixtures of glyceryl monostearate and glyceryl monopalmitate (Myvaplex 600, available from Eastman Fine Chemical Company, Rochester, N.Y.); glyceryl monolinoleate; glyceryl monooleate; mixtures of glyceryl monopalmitate, glyceryl monostearate, monooleate and glyceryl monolinoleate (Myverol Eastman Fine Chemical Company); glyceryl monolinolenate; glyceryl monogadoleate; mixtures of glyceryl monopalmitate, glyceryl monostearate, glyceryl monooleate, glyceryl monolinoleate, glyceryl monolinolenate and glyceryl monogadoleate (Myverol 18-99, Eastman Fine Chemical Company); acetylated glycerides such as distilled acetylated monoglyceride (Myvacet 5-07, 7-07 and 9-45, Eastman Fine Chemical Company); mixtures of propylene glycol monoesters, distilled monoglyceride sodium stearoyl lactylate and silicon dioxide (Myvatex TL, Eastman Fine Chemical Company); mixtures of propylene glycol monoesters, distilled monoglycerides, sodium stearoyl lactylate and silicon dioxide (Myvatex TL, Eastman Fine Chemical Company); d-alpha tocopherol polyethylene glycol 1000 succinate (Vitamin E TPGS, Eastman Fine Chemical Company); mixtures of mono- and diglyceride esters; calcium stearoyl lactylate; ethoxylated mono- and di-glycerides; lactated mono- and di-glycerides; lactylate carboxylic acid esters of glycerol and propylene glycol; lactylic esters of long chain carboxylic acids; polyglycerol esters of long chain carboxylic acids; propylene glycol mono- and di-esters of long chain carboxylic acids; sodium stearoyl lactylate; sorbitan monostearate; sorbitan monooleate; other sorbitan esters of long chain carboxylic acids; succinylated monoglycerides, stearyl monoglyceryl citrate; stearyl heptanoate; cetyl esters of waxes; stearyl octanoate; C 10 -C 30 cholesteroulavosterol esters; and sucrose long chain carboxylic acid esters. Glycerides also include, but are not limited to, at least one of triglyceryl esters such as glyceryl distearate, glyceryl tristearate, glyceryl monostearate, glyceryl dipalmitate, glyceryl tripalmitate, glyceryl monolaurate, glyceryl didocosanoate, glyceryl tridocosanoate, glyceryl monodocosanoate glyceryl monocaprate, glyceryl dicaprate, glyceryl tricaprate, glyceral monomyristate, glyceryl dimyristate, glyceryl trimyristate, glyceryl monodecenoate, glyceryl didecenoate and glyceryl tridecenoate. Glyceride materials can be used singly or in combination with other such agents.

Injectable systems comprising nanoparticles, microcapsules or microspheres of a diameter on the order of about 0.001µm to about 1000µm offer advantages over other delivery systems since such systems inherently are flexible in the design of the duration and rate of separate drug release by selection of microcapsule size, drug loading and dosage administered. In addition, such microcapsules can be sterilized successfully by means such as gamma irradiation or ethylene oxide.

Microspheres and microcapsules are vehicles or systems comprising a polymeric wall that encloses a liquid or solid core. The microshpere wall usually does not react with the core material; however, it is designed to provide sufficient strength to enable normal handling without rupture while being sufficiently thin to allow a high core to wall volume ratio. The sphere/capsule contents remain within the wall until released by diffusion or other means that dissolve, melt, break, rupture or remove the material contained within the sphere/capsule. Preferably, the sphere/capsule wall can be made to degrade and decompose in suitable environments, thus allowing diffusion of the core material through the capsule wall to provide for its slow, sustained delivery.

The mechanism of release in biodegradable microspheres is a combination of drug diffusion and polymer biodegradation. Therefore, the rate and duration of release are determined by microsphere size, drug content and quality, and polymer parameters such as crystallinity, molecular weight and composition. In particular, adjustment in the amount of drug released is generally achieved by modification of wall thickness, diameter, or both.

Moreover, alternative delivery systems based on biodegradable polymers and that are suitable for use in accordance with the present invention, for example, fibers or filaments comprising the active agents, also are contemplated as being within the scope of the present invention when containing effective amounts of the histamine H4 receptor antagonist.

An alternate approach for the single-dose delivery of the histamine H4 receptor antagonist involves the use of biodegradable polymers, such as the ones described above, in the form of a film. Such films may be produced by spraying or discharging dispersed liquid droplets containing the biopolymer and the histamine H4 receptor antagonist in a suitable carrier from a pressurized container onto the targeted site.

Such films, fibers and particles can be prepared by a variety of processes known to those skilled in the art. Such processes include, but are not limited to, spinning disc, solution/precipitation processes, and supercritical fluid processes.

Another approach for the single-dose delivery of the histamine H4 receptor antagonist, in accordance with the present invention, involves the use of liposomes and other lipid-based delivery systems to encapsulate the active agent in multilamellar vesicles (or liposomes). In a typical procedure, a liposome-forming powdered lipid mixture is added to the desired quantity of active agent in aqueous solution, e.g. phosphate buffered saline, to form a suspension. After a suitable hydration period, the hydrated suspension then is autoclaved to provide the liposome-active agent preparations.

The composition of the liposome is usually a combination of phospholipids, particularly high-phase-transition-temperature phospholipids, usually in combination with steroids, especially cholesterol. Other phospholipids or other lipids may also be used. The physical characteristics of liposomes depend on pH, ionic strength, and the presence of divalent cations. Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Particularly useful are diacylphosphatidylglycerols, where the lipid moiety contains from 14-18 carbon atoms, particularly from 16-18 carbon atoms, and is saturated. Illustrative phospholipids include egg phosphatidylcholine, dipalmitoylphosphatidylcholine and distearoylphosphatidylcholine.

A lipid mixture suitable for formation of liposomes may be prepared from L-alpha-distearoyl phosphatidylcholine and cholesterol dissolved in chloroform, to which alpha-tocopherol is added. Other compositions and methods for formation of liposomes also would be useful for this purpose and will be apparent to those skilled on the art once having the benefit of the present disclosure.

Other lipid-based delivery systems also are contemplated for use in this invention. One useful system includes lipid foams such as those available under the tradename DEPOFOAM (SkyPharama, Inc., San Diego, CA), which are extended-release formulations comprising spherical particles bounded by a single bilayer lipid membrane, each containing numerous nonconcentric aqueous chambers which encapsulate the active ingredient. Such lipid particles are made from nontoxic lipids identical to those found in cell membranes.

Another suitable approach for single dose delivery of the histamine H4 receptor antagonist in accordance with the present invention involves the use of crystalloid and so-called viscous instillates. Crystalloids are known in the art as water-soluble crystalline substances, e.g. NaCl, capable of diffusing through a semi-permeable membrane. Solutions of crystalloids, such as saline, are known as crystalloids, crystalloid solutions or crystalloid instillates. Crystalloid instillates include, but are not limited to, lactated Ringer's solution, saline and phosphate buffered saline. In the case of viscous instillates, high-molecular-weight carriers used in admixture with the active agents include, but are not limited to, dextrans and cyclodextrans; hydrogels; cross-linked viscous materials, including viscoelastics and cross-linked viscoelastics; carboxymethylcellulose; poly(saccharide)s; hyaluronic acid; cross-linked hyaluronic acid and hyaluronic acid compounded with orthoesters.

In addition, methods of the present invention further comprise the co-administration of at least one histamine H4 receptor antagonist with an additional therapeutic agent that is useful in the treatment of post-operative adhesions, or useful in the treatment of associated diseases (e.g. as an adjunct with chemotherapeutic drugs), in an amount effective to provide the therapeutic effect intended by administration of the second therapeutic agent. The additional therapeutic agent may be co-administered separately with the at least one histamine H4 receptor antagonist or combined in a pharmaceutical composition according to the invention. In an exemplary embodiment, additional therapeutic agents are those that are known or discovered to be effective in the treatment of post-operative adhesions or inflammation, such as another histamine H₄ receptor modulator or a compound active against another target associated with the condition. The combination may serve to increase efficacy (e.g., by including in the combination a compound potentiating the potency or effectiveness of a histamine H4 receptor antagonist), decrease one or more side effects, or decrease the required dose of the active agent according to the invention.

The additional therapeutic agents that may be used in combination with the histamine H4 receptor antagonist may fall in the general classes of anti-platelet, anti-fibrotic, anti-inflammatory, anti-proliferative, and/or inhibitors of collagen synthesis. These include, but are not limited to, Urokinase, the non-glycosylated deletion mutein of tissue plasminogen activator available under the tradename RETAVASE (Boehringer Manheim, Indianapolis, IN), pharmaceutical preparations containing abciximab for the prevention and treatment of diseases of the circulatory system available under the tradename REOPRO (Eli Lilly and Company, Indianapolis IN), Clopidogrel Bisulfate, available under the tradename PLAVIX (Sanofi-Synthelabo, Paris, France), pharmaceutical preparations containing imatinib mesylate for use in the field of oncology available under the tradename GLEEVEC (Novartis AG, Basel Switzerland), Triamcinolone Acetonide, Tepoxalin, Pirfenidone, collagenase, anti-CTGF, tyrosine kinase inhibitors, prolyl hydroxylase inhibitors, lysly oxidase inhibitors, C-proteinase inhibitors, N-proteinase inhibitors, TGFβ inhibitors such as Tamoxifen, HMG-CoA Reductase inhibitors such as Lovastatin, COX-1 and/or COX-2 inhibitors such as Ibuprofen, Nimesulide, pharmaceutical preparation containing vofecoxib for the treatment of arthritis available under the tradename VIOXX (Merck & Co., Inc. Whitehouse Station NJ), pharmaceuticals in the nature of anti-inflammatory analgesics containing celecoxib available under the tradename CELEBREX (G.D. Searle & Co., Skokie IL), pharmaceutical preparations containing valdecoxib available under the tradename BEXTRA (Pharmacia & Upjohn Co., North Peapackn NJ), Calcium ion inhibitors such as Amlodipine, Nifedipine, pharmaceuticals such as verapamil used in the treatment of hypertension, iron chelators such as deferoxamine available under the tradename DESFERAL (Novartis AG, Basel Switzerland), antibiotics such as Clarithromycin and Ciprofloxin retinoids such as Tretinoin and Retinoic Acid, chymase inhibitors, and 9-methyl-3-(1H-tetrazol-5-yl)-4H-pyrido[1,2-α]pyrimidin-4-one potassium, also known as Pemirolast. When used in combination with the histamine H4 receptor antagonist, the therapeutic agents, or drugs, are present in an amount effective to provide the therapeutic effect intended by administration of the therapeutic agent.

For example, a delivery vehicle in the form of a barrier and the histamine H4 receptor antagonist could show greater efficacy if combined with other drugs at the time of surgery or pre-operatively. For example, an anti-fibrotic such as the recombinant plasminogen activator compound available under the tradename RETAVASE (Boehringer Mannheim Corp., Indianapolis, IN) would be delivered to the site at the time of surgery and then a barrier/collagen synthesis inhibitor (such as a histamine H4 receptor antagonist) would be placed onto the site. The combined effect of the plaminogen activator compound limiting the clotting at the surgical site, the barrier limiting the apposition of the tissue surfaces and the histamine H4 receptor antagonist inhibiting collagen synthesis could dramatically reduce adhesions. The additional therapeutic agents also could be given systemically, by a variety of means, prior to, during or after surgery in conjunction with local, non-systemic administration post-operatively.

In one embodiment of the invention, the histamine H4 receptor antagonist is combined with a physical barrier. It is believed that for a combination of the proper physical barrier and the histamine H4 receptor antagonist, an unexpected synergistic effect could be created that yields results better than either the histamine H4 receptor antagonist or barrier used alone. For example, a barrier comprising a polyethylene glycol whose surface properties are antithrombogenic, and therefore could prevent platelet adherence, could prevent some fibrin clotting from occurring. At the same time, the histamine H4 receptor antagonist that affects a later event in the adhesion sequence, e.g. collagen synthesis, could be delivered to the site over an extended period of time. Hence, by affecting more than one adhesion-producing event, the histamine H4 receptor antagonist/barrier combination will have efficacy that is greater than the sum of the histamine H4 receptor antagonist and barrier. Other barriers also could exhibit such effects in combination with the histamine H4 receptor antagonist.

As another example, hyaluronic acids have been proposed to reduce cell proliferation (anti-proliferative) as well as being excellent coatings that would provide a lubricious surface between apposed tissue surfaces. The body excretes hyaluronic acid for just such a purpose, i.e., articulating surfaces-joints. Such a barrier could be, for example, a polymeric carboxymethylcellulose gel that is hydrophilic, so that it adheres to the tissues of the site, and has excellent biocompatibility, so that it does not cause an inflammatory response that could elicit collagen synthesis. Combined with the histamine H4 receptor antagonist, a hyaluronic acid barrier could be more effective than the histamine H4 receptor antagonist or itself.

Other barriers include, but are not limited to, various derivatives of hyaluronic acids (salts such as iron, sodium; esters such as benzyl); cellulosics derivatives (oxidized regenerated; methyl; ethyl; hydroxypropyl); collagens; polyethylene glycols (including in-situ crosslinked); pluronics; chitin, chitosans; dextrans; glucoses; carbohydrates; gelatins; glycosaminoglycans; polyacrylamides; polyvinyl pyrrolidones; polyvinyl alcohols; polymethyacrylics; aliginates; starches; polypeptides; and any other water soluble polymer and blends thereof. Such polymers could also be copolymerized or blended with hydrolyzable or enzymatically degradable polymers such as polylactones, polyoxaesters, polyalkylene diglycolates, and glyceride containing polymers, and copolymer and blends thereof. Barriers also could be non-absorbable barriers such as polytetrafluoroethylene. The histamine H4 receptor antagonist and/or other therapeutics of the present invention may be covalently or non-covalently (e.g., ionically) bound to such a barrier, or it may simply be dispersed therein.

It also should be known that the delivery vehicles described herein not only may include a barrier such as a gel that would deliver the drug locally, but also could include delivery of the drug(s) via other local administration methods such as an osmotic pump.

A "pharmaceutically acceptable excipient" refers to a substance that is non-toxic, biologically tolerable, and otherwise biologically suitable for administration to a subject, such as an inert substance, added to a pharmacological composition or otherwise used as a vehicle, carrier, or diluent to facilitate administration of a agent and that is compatible therewith. Examples of excipients include calcium carbonate, calcium phosphate, various sugars and types of starch, cellulose derivatives, gelatin, vegetable oils, and polyethylene glycols.

Delivery forms of the pharmaceutical compositions containing one or more dosage units of the active agents may be prepared using suitable pharmaceutical excipients and compounding techniques known or that become available to those skilled in the art. The compositions may be administered in the inventive methods by a suitable route of delivery, e.g., oral, parenteral, rectal, topical, or ocular routes, or by inhalation.

The preparation may be in the form of tablets, capsules, sachets, dragees, powders, granules, lozenges, powders for reconstitution, liquid preparations, or suppositories. Preferably, the compositions are formulated for intravenous infusion, topical administration, or oral administration.

For oral administration, the active agents of the invention can be provided in the form of tablets or capsules, or as a solution, emulsion, or suspension. To prepare the oral compositions, the active agents may be formulated to yield a dosage of, e.g., from about 0.05 to about 50 mg/kg daily, or from about 0.05 to about 20 mg/kg daily, or from about 0.1 to about 10 mg/kg daily.

Oral tablets may include the active ingredient(s) mixed with compatible pharmaceutically acceptable excipients such as diluents, disintegrating agents, binding agents, lubricating agents, sweetening agents, flavoring agents, coloring agents and preservative agents.

Suitable inert fillers include sodium and calcium carbonate, sodium and calcium phosphate, lactose, starch, sugar, glucose, methyl cellulose, magnesium stearate, mannitol, sorbitol, and the like. Exemplary liquid oral excipients include ethanol, glycerol, water, and the like. Starch, polyvinyl-pyrrolidone (PVP), sodium starch glycolate, microcrystalline cellulose, and alginic acid are exemplary disintegrating agents. Binding agents may include starch and gelatin. The lubricating agent, if present, may be magnesium stearate, stearic acid or talc. If desired, the tablets may be coated with a material such as glyceryl monostearate or glyceryl distearate to delay absorption in the gastrointestinal tract, or may be coated with an enteric coating.

Capsules for oral administration include hard and soft gelatin capsules. To prepare hard gelatin capsules, active ingredient(s) may be mixed with a solid, semisolid, or liquid diluent. Soft gelatin capsules may be prepared by mixing the active ingredient with water, an oil such as peanut oil or olive oil, liquid paraffin, a mixture of mono and di-glycerides of short chain fatty acids, polyethylene glycol 400, or propylene glycol.

Liquids for oral administration may be in the form of suspensions, solutions, emulsions or syrups or may be lyophilized or presented as a dry product for reconstitution with water or other suitable vehicle before use. Such liquid compositions may optionally contain: pharmaceutically-acceptable excipients such as suspending agents (for example, sorbitol, methyl cellulose, sodium alginate, gelatin, hydroxyethylcellulose, carboxymethylcellulose, aluminum stearate gel and the like); non-aqueous vehicles, e.g., oil (for example, almond oil or fractionated coconut oil), propylene glycol, ethyl alcohol, or water; preservatives (for example, methyl or propyl p-hydroxybenzoate or sorbic acid); wetting agents such as lecithin; and, if desired, flavoring or coloring agents.

The active agents of this invention may also be administered systemically by non-oral routes. For example, compositions may be formulated for rectal administration as a suppository. For parenteral use, including intravenous, intramuscular, intraperitoneal, or subcutaneous routes, the agents of the invention may be provided in sterile aqueous solutions or suspensions, buffered to an appropriate pH and isotonicity or in parenterally acceptable oil. Suitable aqueous vehicles include Ringer's solution and isotonic sodium chloride. Such forms may be presented in unit-dose form such as ampules or disposable injection devices, in multi-dose forms such as vials from which the appropriate dose may be withdrawn, or in a solid form or pre-concentrate that can be used to prepare an injectable formulation. Illustrative infusion doses range from about 1 to 1000 µg/kg/minute of agent admixed with a pharmaceutical carrier over a period ranging from several minutes to several days.

For topical administration, the agents may be mixed with a pharmaceutical carrier at a concentration of about 0.1% to about 10% of drug to vehicle. Another mode of administering the agents of the invention may utilize a patch formulation to affect transdermal delivery.

Active agents may alternatively be administered in methods of this invention by inhalation, via the nasal or oral routes, e.g., in a spray formulation also containing a suitable carrier.

The invention may be better understood with reference to the accompanying examples, which are intended to be illustrative only and should not be viewed as in any sense limiting the scope of the invention, which is defined hereinafter in the accompanying claims.

### Examples

Multiple studies to confirm the efficacy of histamine H4 receptor antagonists in the reduction or inhibition of adhesion formation after peritoneal surgery were performed using a sidewall adhesion model.

### Peritoneal Sidewall Model

Animals: Twenty eight female New Zealand White rabbits, 2.4-2.7 kg, were purchased from Irish Farms and quarantined for at least 2 days prior to use. The rabbits were housed on a 12:12 light:dark cycle with food and water available ad libitum.

Rabbits were anesthetized with a mixture of 55 mg/kg ketamine hydrochloride and 5 mg/kg Rompum intramuscularly. Following preparation for sterile surgery, a midline laparotomy was performed. A polyethylene catheter (Clay Adams polyethylene tubing PE-60 ID 0.76 mm (0.030") OD 1.22 mm (0.048")) was introduced into the peritoneal cavity and sutured to the sidewall with 5-0 Ethilon. For local administration, an Alzet miniosmotic pump (10 µl/hour, 2 ml, Model 2ML1 from Durect), filled with one dosage level or placebo (water for injection) control at 10 µl/hour over 7 days starting with the day of surgery was placed in the subcutaneous space. The catheter was then attached to the pump and the midline muscle incision was closed around the catheter. The cecum and bowel were exteriorized and digital pressure was exerted to create subserosal hemorrhages over all surfaces. The damaged intestine was then lightly abraded with 4" x 4" 4-ply sterile gauze until punctate bleeding was observed. The cecum and bowel was then returned to their normal anatomic position. A 5 x 3 cm area of peritoneum and transversus abdominous muscle was removed on the right lateral abdominal wall. The incision was closed in two layers with 3-0 Vicryl (Ethicon, Somerville, NJ). The pump was replaced at day 7.

Twenty-one days after surgery, rabbits were sacrificed by intravenous EuthaSol. Adhesion formation at the site of sidewall injury was assessed by examination to determine the percentage of the area of the sidewall injury involved in adhesions. The percentage of a surface of the sidewall involved in adhesions to various organs are given in the tables below to quantify the overall adhesion score. In addition, tenacity of the adhesion formed was scored using a system as follows:
0 = No adhesions;
1 = mild, easily dissectible adhesions;
2 = moderate adhesions; non-dissectible, does not tear organ;
3 = dense adhesions; non-dissectible, tears when removed.

The difference in adhesion formation between the control and treated groups were analyzed by Student's t-test. A reduction in the area and the tenacity of the adhesions would be considered beneficial and efficacious.

In order to evaluate the pharmacokinetics, 1 ml blood samples were taken from each animal on days 0 (before surgery) and 7, after placement of the pumps. Blood was collected in heparinized tubes, centrifuged to pellet the cells, and the supernatant (plasma) stored at -80°C.

General observations were conducted on each animal (appearance, behavior, clinical signs, symptoms, moribundity, mortality, and physical examinations) prior to assignment to study and on the day of necropsy. On the day of necropsy data and observations were recorded with particular attention to inflammation, granulomatous material and adhesion formation at the treatment site. All biocompatibility results were recorded, and the level of reaction was noted (none, mild, moderate, severe).

Non-systemic administration of (5-Chloro-1H-indol-3-yl)-(4-methyl-piperazin-1-yl)-methanone.

The 28 rabbits used in this study were divided into the following treatment groups:
Group 1: No Treatment
Group 2: Vehicle control
Group 3: 5 mg/ml of (5-chloro-1H-indol-3-yl)-(4-methyl-piperazin-1-yl)-methanone

One control animal died on day 10 due to peritonitis. No other rabbits showed any clinical signs. There were no inflammatory or granulomatous reactions noted in the peritoneal cavity. The placebo (Table 2) did not significantly affect area or tenacity of adhesion formation compared with surgical control (Table 1). (5-Chloro-1H-indol-3-yl)-(4-methyl-piperazin-1-yl)-methanone significantly reduced both the area and tenacity of adhesions (Table 3).

**Table 1. Adhesion Scores in Surgical Control Animals**

| **Animal Number** | **Adhesion Area** | **Adhesion Tenacity** |
|---|---|---|
| **1** | 100 | 3 |
| **2** | 100 | 3 |
| **3** | 90 | 2 |
| **4** | 100 | 2 |
| **5** | 100 | 2 |
| **6** | 100 | 3 |
| **Mean/SEM** | 98.3 ± 1.7 | 19.75 ± 1.9* |

| | | |
|---|---|---|
| *This value is the mean of the rank of the tenacity (a non-parametric measure). | | |

**Table 2. Adhesion Scores in Placebo Control Animals**

| **Animal Number** | **Adhesion Area** | **Adhesion Tenacity** |
|---|---|---|
| **1** | 100 | 3 |
| **2** | 100 | 3 |
| **3** | 100 | 2 |
| **4** | 100 | 1 |
| **5** | 100 | 1 |
| **6** | 100 | 2 |
| **7** | 100 | 3 |
| **Mean/SEM** | 100 ± 0 | 16.29 ± 3.1* |

| | | |
|---|---|---|
| *This value is the mean of the rank of the tenacity (a non-parametric measure). | | |

**Table 3. Adhesion Scores in Animals Treated with 5 mg/ml (5-Chloro-1H-indol-3-yl)-(4-methyl-piperazin-1-yl)-methanone**

| **Animal Number** | **Adhesion Area** | **Adhesion Tenacity** |
|---|---|---|
| **1** | 80 | 1 |
| **2** | 50 | 1 |
| **3** | 100 | 1 |
| **4** | 100 | 2 |
| **5** | 60 | 1 |
| **6** | 70 | 1 |
| **7** | 40 | 1 |
| **Mean/SEM** | 71.4 ± 8.8 | 6.93 ± 1.4 |

| | | |
|---|---|---|
| *This value is the mean of the rank of the tenacity (a non-parametric measure). | | |

The test compound in another set of experiments was [5-(4,6-dimethyl-1H-benzoimidazol-2-yl)-4-methyl-pyrimidin-2-yl]-[3-(1-methyl-piperidin-4-yl)-propyl]-amine, administered at the doses listed below. The 35 animals used in this study were divided into the following treatment groups:
Group 1: Surgical Control
   Non-systemic administration (pump):
Group 2: 0.1 mg/ml test compound
Group 3: 1 mg/ml test compound
Group 4: 10 mg/ml test compound
   Systemic administration (delivery by oral gavage on day of surgery and four additional days):
Group 5: 30 mg/kg test compound orally

All animals, except one control (died on day 12), survived until necropsy. Further, in some animals, white precipitate was observed at the end of the tubing. In the animals where the drug was administered by Alzet pump, the efficacy observed was surrounding the end of the tube. This was seen in 1 each of the low and mid dose animals and 3 of the high dose animals. While both area and tenacity appeared reduced at the mid-dose of drug administered by pump, only the area of adhesion reduction was statistically significant.

**Table 1. Adhesion Scores in Placebo Control Animals**

| **Animal Number** | **Adhesion Area** | **Adhesion Tenacity** |
|---|---|---|
| **1** | 100 | 2 |
| **2** | 100 | 1 |
| **3** | 100 | 2 |
| **4** | 100 | 3 |
| **5** | 100 | 2 |
| **6** | 100 | 2 |
| **7** | 80 | 2 |
| **Mean/SEM** | 97.1 ± 2.86 | 20.1 ± 3.37* |

| | | |
|---|---|---|
| *This value is the mean of the rank of the tenacity (a non-parametric measure). | | |

**Table 2. Adhesion Scores in 0.1 mg/ml Test Compound**

| **Animal Number** | **Adhesion Area** | **Adhesion Tenacity** |
|---|---|---|
| **1** | 70 | 2 |
| **2** | 70 | 1 |
| **3** | 100 | 2 |
| **4** | 100 | 2 |
| **5** | 80 | 2 |
| **6** | 80 | 2 |
| **7** | 100 | 3 |
| **Mean/SEM** | 85.7 ± 5.28 | 20.1 ± 3.37* |

| | | |
|---|---|---|
| *This value is the mean of the rank of the tenacity (a non-parametric measure). | | |

**Table 3. Adhesion Scores in 1 mg/ml Test Compound**

| **Animal Number** | **Adhesion Area** | **Adhesion Tenacity** |
|---|---|---|
| **1** | 60 | 2 |
| **2** | 60 | 1 |
| **3** | 80 | 2 |
| **4** | 60 | 1 |
| **5** | 80 | 2 |
| **6** | 80 | 2 |
| **7** | 70 | 1 |
| **Mean/SEM** | 70 ± 10 | 13.9 ± 3.38* |

| | | |
|---|---|---|
| *This value is the mean of the rank of the tenacity (a non-parametric measure). | | |

**Table 4. Adhesion Scores in 10 mg/ml Test Compound**

| **Animal Number** | **Adhesion Area** | **Adhesion Tenacity** |
|---|---|---|
| **1** | 80 | 2 |
| **2** | 80 | 2 |
| **3** | 60 | 2 |
| **4** | 100 | 2 |
| **5** | 90 | 2 |
| **6** | 100 | 2 |
| **7** | 60 | 1 |
| **Mean/SEM** | 81.4 ± 6.34 | 18.3 ± 1.43* |

| | | |
|---|---|---|
| *This value is the mean of the rank of the tenacity (a non-parametric measure). | | |

**Table 5. Adhesion Scores in 30 mg/kg Test Compound**

| **Animal Number** | **Adhesion Area** | **Adhesion Tenacity** |
|---|---|---|
| **1** | 40 | 1 |
| **2** | 70 | 2 |
| **3** | 80 | 1 |
| **4** | 100 | 2 |
| **5** | 60 | 1 |
| **6** | 100 | 3 |
| **7** | 100 | 3 |
| **Mean/SEM** | 78.6 ± 8.85 | 17.6 ± 5.27* |

| | | |
|---|---|---|
| *This value is the mean of the rank of the tenacity (a non-parametric measure). | | |

While the fundamental novel features of the invention have been shown and described, it will be understood that various omissions, substitutions and changes in the form and details illustrated may be made by those skilled in the art without departing from the spirit of the invention. It is the intention, therefore, to be limited only as indicated by the scope of the following claims.

### Embodiments :

1. A method for the inhibition of post-operative adhesion formation in a body between tissue surfaces in a body cavity having been subjected to a surgical procedure, comprising administering an effective amount of at least one histamine H4 receptor antagonist to the tissue surfaces in the body cavity, wherein the at least one histamine H4 receptor antagonist is selected from the group consisting of:
   a) benzoimidazolyl and benzoxazolyl amides and thioamides;
   b) 8-membered bicyclic aromatic amides and thioamides;
   c) 9-membered bicyclic aromatic amides, thioamides, and imides;
   b) amino-substituted quinoxalines;
   d) 2-arylbenzimidazole ether compounds;
   e) 2-arylimidazole ether compounds;
   f) benzoimidazol-2-yl pyridines;
   g) indolyl and benzimidazolyl pyrrolidinyl amides;
   h) benzoimidazol-2-yl pyrimidines and pyrazines;
   i) benzofuro- and benzothienopyrimidines;
   j) 2-aminopyrimidines;
   k) aryl-substitued pyridines and triazines;
   l) thieno- and furo-pyrimidines;
   m) bicyclic heteroaryl-substituted imidazoles;
      pharmaceutically acceptable salts, hydrates, solvates and mixtures of any of said H4 receptor antagonists.
2. The method of embodiment 1 wherein the at least one histamine H4 receptor antagonist is administered by a delivery vehicle suitable for use in the local, non-systemic administration of a therapeutic agent to the tissue surfaces.
3. The method of embodiment 2 wherein the delivery vehicle is at least one of nanoparticles, microcapsules, microspheres, barriers, liposomes, lipid foams, solutions, compositions, osmotic pumps, fibers, filaments, gels and films.
4. The method of embodiment 3 wherein the barrier is absorbable.
5. The method of embodiment 1 wherein the at least one histamine H4 receptor antagonist is administered in combination with an additional therapeutic agent, the additional therapeutic agent administered in an amount effective to provide the therapeutic effect intended by administration of the additional therapeutic agent.
6. The method of embodiment 5 wherein the additional therapeutic agent is at least one of an anti-platelet, an anti-fibrotic, an anti-inflammatory, an anti-proliferative and an agent that inhibits collagen synthesis.
7. The method of embodiment 1 wherein the at least one histamine H4 receptor antagonist is selected from the group consisting of: benzoimidazolyl and benzoxazolyl amides and thioamides, benzoimidazol-2-yl pyrimidines and pyrazines, pharmaceutically acceptable salts, hydrates, solvates and mixtures of any of said H4 receptor antagonists.
8. The method of embodiment 1 wherein the at least one histamine H4 receptor antagonist is selected from the group consisting of: (5-chloro-1H-indol-3-yl)-(4-methyl-piperazin-1-yl)-methanone, [5-(4,6-dimethyl-1H-benzoimidazol-2-yl)-4-methyl-pyrimidin-2-yl]-[3-(1-methyl-piperidin-4-yl)-propyl]-amine, a pharmaceutically acceptable salt, a hydrate, a solvate, and a mixture of any of said H4 receptor antagonists.
9. The method of embodiment 1 wherein the at least one histamine H4 receptor antagonist is administered in a single dose.
10. The method of embodiment 1 wherein the at least one histamine H4 receptor antagonist is administered by sustained release.
11. The method of embodiment 1 wherein the at least one histamine H4 receptor antagonist is administered by burst/sustained release.
12. The method of embodiment 1 wherein the at least one histamine H4 receptor antagonist is administered at a level of from about 0.001 milligram per kilogram of the body to about 200 milligram per kilogram of the body.
13. The method of embodiment 1 further comprising administering the at least one histamine H4 receptor antagonist systemically to the body prior to the surgical procedure.
14. A delivery vehicle suitable for local, non-systemic administration of a drug directly to tissue within a body cavity having been subjected to a surgical procedure, the vehicle comprising an effective amount of at least one histamine H4 receptor antagonist, wherein the at least one histamine H4 receptor antagonist is selected from the group consisting of:
   a) benzoimidazolyl and benzoxazolyl amides and thioamides;
   b) 8-membered bicyclic aromatic amides and thioamides;
   c) 9-membered bicyclic aromatic amides, thioamides, and imides;
   b) amino-substituted quinoxalines;
   d) 2-arylbenzimidazole ether compounds;
   e) 2-arylimidazole ether compounds;
   f) benzoimidazol-2-yl pyridines;
   g) indolyl and benzimidazolyl pyrrolidinyl amides;
   h) benzoimidazol-2-yl pyrimidines and pyrazines;
   i) benzofuro- and benzothienopyrimidines;
   j) 2-aminopyrimidines;
   k) aryl-substitued pyridines and triazines;
   l) thieno- and furo-pyrimidines;
   m) bicyclic heteroaryl-substituted imidazoles;
      pharmaceutically acceptable salts, hydrates, solvates and mixtures of any of said H4 receptor antagonists.
15. The delivery vehicle of embodiment 14, wherein said vehicle comprises at least one of nanoparticles, microcapsules, microspheres, barriers, liposomes, lipid foams, solutions, compositions, osmotic pumps, fibers, filaments, gels and films.
16. The delivery vehicle of embodiment 15 comprising a polymer that is at least one polymer in the group of poloxamers, poly(orthoester)s, poly(vinyl alcohol)s, poly(anhydride)s, poly(methacrylate)s, poly(methacryladmide)s, anionic carbohydrate polymers, poly(hydroxybutyric acid)s, polyacetals, poly(1-lactide), poly(dl-lactide), poly(dl-lactide-co-glycolide)s, poly(1-lactide-co-glycolide)s, poly(e-caprolactone), polyglycolide, poly(p-dioxanone)s, poly(trimethylene carbonate), poly(alkylene diglycolate)s, poly(oxaester)s, poly(oxaamide)s and glyceride polymers.
17. The delivery vehicle of embodiment 15 wherein the liposome is at least one of L-alpha-distearoyl phosphatidylcholine, phosphatidylcholine, dipalmitoylphosphatidylcholine, and distearoylphosphatidylcholine.
18. The delivery vehicle of embodiment 15 wherein the solution comprises a crystalloid instillate, and said crystalloid instillate comprises at least one of phosphate buffered saline, saline, and lactated Ringer's solution.
19. The delivery vehicle of embodiment 15 wherein the solution comprises viscous instillate, said viscous instillate comprising at least one carrier in the group of dextrans, cyclodextrans, hydrogels, carboxymethylcellulose, poly(saccharide)s, hyaluronic acids, crosslinked hyaluronic acids and chondroitin sulfates.
20. The delivery vehicle of embodiment 15 wherein the barrier is absorbable.
21. The delivery vehicle of embodiment 20 wherein the absorbable barrier comprises at least one of hyaluronic acids, cellulosics derivatives, collagens, polyethylene glycols, pluronics, chitin, chitosans, dextrans, glucoses, carbohydrates, gelatins, glycosaminoglycans, polyacrylamides, polyvinyl pyrrolidones, polyvinyl alcohols, polymethyacrylics, aliginates, starches and polypeptides.
22. The delivery vehicle of embodiment 14 further comprising an additional therapeutic agent in an amount effective to provide the therapeutic effect intended by administration of the additional therapeutic agent.
23. The delivery vehicle of embodiment 22 wherein the additional therapeutic agent is at lest one of an anti-platelet, an anti-fibrotic, an anti-inflammatory, an anti-proliferative, and an agent that inhibits collagen synthesis.
24. The delivery vehicle of embodiment 14 wherein the vehicle provides for single dose administration of the at least one histamine H4 receptor antagonist.
25. The delivery vehicle of embodiment 14 wherein the vehicle provides for sustained release of the at least one histamine H4 receptor antagonist.
26. The method of embodiment 14 wherein the vehicle provides for burst/sustained release of the at least one histamine H4 receptor antagonist.
27. The delivery vehicle of embodiment 14 comprising from about 0.001 milligram of the at least one histamine H4 receptor antagonist per kilogram of the body to about 200 milligrams of the at least one histamine H4 receptor antagonist per kilogram of the body.
28. A composition suitable for local, non-systemic administration of a drug directly to tissue within a body cavity having been subjected to a surgical procedure, the composition comprising an effective amount of at least one histamine H4 receptor antagonist, wherein the at least one histamine H4 receptor antagonist is selected from the group consisting of:
   a) benzoimidazolyl and benzoxazolyl amides and thioamides;
   b) 8-membered bicyclic aromatic amides and thioamides;
   c) 9-membered bicyclic aromatic amides, thioamides, and imides;
   b) amino-substituted quinoxalines;
   d) 2-arylbenzimidazole ether compounds;
   e) 2-arylimidazole ether compounds;
   f) benzoimidazol-2-yl pyridines;
   g) indolyl and benzimidazolyl pyrrolidinyl amides;
   h) benzoimidazol-2-yl pyrimidines and pyrazines;
   i) benzofuro- and benzothienopyrimidines;
   j) 2-aminopyrimidines;
   k) aryl-substitued pyridines and triazines;
   l) thieno- and furo-pyrimidines;
   m) bicyclic heteroaryl-substituted imidazoles;
      pharmaceutically acceptable salts, hydrates, solvates, and mixtures of said histamine H4 receptor antagonists.
29. The composition of embodiment 28, further comprising a carrier suitable for local, non-systemic administration of the at least one histamine H4 receptor antagonist.
30. The composition of embodiment 29 wherein the carrier is at least one of nanoparticles, microcapsules, microspheres, barriers, liposomes, lipid foams, solutions, osmotic pumps, fibers, filaments, gels and films.
31. The composition of embodiment 29 wherein the carrier comprises a polymer that is at least one polymer in the group of poloxamers, poly(orthoester)s, poly(vinyl alcohol)s, poly(anhydride)s, poly(methacrylate)s, poly(methacryladmide)s, anionic carbohydrate polymers, poly(hydroxybutyric acid)s, polyacetals, poly(1-lactide), poly(dl-lactide), poly(dl-lactide-co-glycolide)s, poly(1-lactide-co-glycolide)s, poly(e-caprolactone), polyglycolide, poly(p-dioxanone)s, poly(trimethylene carbonate), poly(alkylene diglycolate)s, poly(oxaester)s, poly(oxaamide)s and glyceride polymers.
32. The composition of embodiment 28 wherein the composition provides for single dose administration of the at least one histamine H4 receptor antagonist.
33. The composition of embodiment 28 wherein the composition provides for sustained release of the at least one histamine H4 receptor antagonist.
34. The composition of embodiment 28 wherein the composition provides for burst/sustained release of the at least one histamine H4 receptor antagonist.
35. The composition of embodiment 28 comprising from about 0.001 milligram of the at least one histamine H4 receptor antagonist per kilogram of the body to about 200 milligrams of the at least one histamine H4 receptor antagonist per kilogram of the body.
36. The delivery vehicle of embodiment 30 wherein the liposome is at least one of L-alpha-distearoyl phosphatidylcholine, phosphatidylcholine, dipalmitoylphosphatidylcholine, and distearoylphosphatidylcholine.
37. The delivery vehicle of embodiment 30 wherein the solution comprises a crystalloid instillate, and said crystalloid instillate is at least one of phosphate buffered saline, saline and lactated Ringer's solution.
38. The delivery vehicle of embodiment 30 wherein the solution comprises viscous instillate comprising a carrier, and said carrier comprises at least one of dextrans, cyclodextrans, hydrogels, carboxymethylcellulose, poly(saccharide)s, hyaluronic acids, crosslinked hyaluronic acids and chondroitin sulfates.
39. The delivery vehicle of embodiment 30 wherein the barrier is absorbable.
40. The delivery vehicle of embodiment 39 wherein the absorbable barrier is at least one of hyaluronic acids, cellulosics derivatives, collagens, polyethylene glycols, pluronics, chitin, chitosans, dextrans, glucoses, carbohydrates, gelatins, glycosaminoglycans, polyacrylamides, polyvinyl pyrrolidones, polyvinyl alcohols, polymethyacrylics, aliginates, starches and polypeptides.
41. The delivery vehicle of embodiment 28 further comprising an additional therapeutic agent in an amount effective to provide the therapeutic effect intended by administration of the additional therapeutic agent.
42. The delivery vehicle of embodiment 41 wherein the additional therapeutic agent is at least one of an anti-platelet, an anti-fibrotic, an anti-inflammatory, an anti-proliferative and an agent that inhibits collagen synthesis.
43. A method for the inhibition of post-operative adhesion formation in a body between tissue surfaces in a body cavity having been subjected to a surgical procedure comprising administering an effective amount of at least one histamine H4 receptor antagonist systemically, wherein the at least one histamine H4 receptor antagonist is selected from the group consisting of:
   a) benzoimidazolyl and benzoxazolyl amides and thioamides;
   b) 8-membered bicyclic aromatic amides and thioamides;
   c) 9-membered bicyclic aromatic amides, thioamides, and imides;
   b) amino-substituted quinoxalines;
   d) 2-arylbenzimidazole ether compounds;
   e) 2-arylimidazole ether compounds;
   f) benzoimidazol-2-yl pyridines;
   g) indolyl and benzimidazolyl pyrrolidinyl amides;
   h) benzoimidazol-2-yl pyrimidines and pyrazines;
   i) benzofuro- and benzothienopyrimidines;
   j) 2-aminopyrimidines;
   k) aryl-substitued pyridines and triazines;
   l) thieno- and furo-pyrimidines;
   m) bicyclic heteroaryl-substituted imidazoles;
      pharmaceutically acceptable salts, hydrates, solvates and mixtures of said histamine H4 receptor antagonists.
44. The method of embodiment 43, wherein the administering is done before surgery.
45. The method of embodiment 43, wherein the administering is done after surgery.

## Claims

1. A histamine H4 receptor antagonist for use in a method for the inhibition of post-operative adhesion formation in a body between tissue surfaces in a body cavity having been subjected to a surgical procedure, comprising administering an effective amount of at least one histamine H4 receptor antagonist to the tissue surfaces in the body cavity, wherein the at least one histamine H4 receptor antagonist is selected from the group consisting of:
benzoimidazol-2-yl pyrimidines and pyrazines;
pharmaceutically acceptable salts, hydrates, solvates and mixtures of any of said H4 receptor antagonists.

2. The histamine H4 receptor antagonist as claimed in claim 1 for use in the method according to claim 1 wherein the at least one histamine H4 receptor antagonist is administered by a delivery vehicle suitable for use in the local, non-systemic administration of a therapeutic agent to the tissue surfaces.

3. The histamine H4 receptor antagonist as claimed in claim 2 for use in the method according to claim 2 wherein the delivery vehicle is at least one of nanoparticles, microcapsules, microspheres, barriers, liposomes, lipid foams, solutions, compositions, osmotic pumps, fibers, filaments, gels and films.

4. The histamine H4 receptor antagonist as claimed in claim 1 for use in the method according to claim 1 wherein the at least one histamine H4 receptor antagonist is administered in combination with an additional therapeutic agent, the additional therapeutic agent administered in an amount effective to provide the therapeutic effect intended by administration of the additional therapeutic agent.

5. The histamine H4 receptor antagonist as claimed in claim 4 for use in the method according to claim 4 wherein the additional therapeutic agent is at least one of an anti-platelet, an anti-fibrotic, an anti-inflammatory, an anti-proliferative and an agent that inhibits collagen synthesis.

6. The histamine H4 receptor antagonist as claimed in claim 1 for use in the method according to claim 1 wherein the at least one histamine H4 receptor antagonist is selected from the group consisting of: [5-(4,6-dimethyl-1H-benzoimidazol-2-yl)-4-methyl-pyrimidin-2-yl]-[3-(1-methyl-piperidin-4-yl)-propyl]-amine, a pharmaceutically acceptable salt, a hydrate, a solvate, and a mixture of any of said H4 receptor antagonists.

7. The histamine H4 receptor antagonist as claimed in claim 1 for use in the method according to claim 1 wherein the at least one histamine H4 receptor antagonist is administered in a single dose.

8. The histamine H4 receptor antagonist as claimed in claim 1 for use in the method according to claim 1 wherein the at least one histamine H4 receptor antagonist is administered by sustained release.

9. The histamine H4 receptor antagonist as claimed in claim 1 for use in the method according to claim 1 wherein the at least one histamine H4 receptor antagonist is administered by burst/sustained release.

10. The histamine H4 receptor antagonist as claimed in claim 1 for use in the method according to claim 1 wherein the at least one histamine H4 receptor antagonist is administered at a level of from about 0.001 milligram per kilogram of the body to about 200 milligram per kilogram of the body.

11. The histamine H4 receptor antagonist as claimed in claim 1 for use in the method according to claim 1 further comprising administering the at least one histamine H4 receptor antagonist systemically to the body prior to the surgical procedure.

12. A delivery vehicle suitable for local, non-systemic administration of a drug directly to tissue within a body cavity having been subjected to a surgical procedure, the vehicle comprising an effective amount of at least one histamine H4 receptor antagonist, wherein the at least one histamine H4 receptor antagonist is selected from the group consisting of:
benzoimidazol-2-yl pyrimidines and pyrazines;
pharmaceutically acceptable salts, hydrates, solvates and mixtures of any of said H4 receptor antagonists.

13. A composition suitable for local, non-systemic administration of a drug directly to tissue within a body cavity having been subjected to a surgical procedure, the composition comprising an effective amount of at least one histamine H4 receptor antagonist, wherein the at least one histamine H4 receptor antagonist is selected from the group consisting of:
benzoimidazol-2-yl pyrimidines and pyrazines;
pharmaceutically acceptable salts, hydrates, solvates, and mixtures of said histamine H4 receptor antagonists.

14. The composition as claimed in claim 13, further comprising a carrier suitable for local, non-systemic administration of the at least one histamine H4 receptor antagonist.

15. The delivery vehicle as claimed in claim 12 or the composition as claimed in claim 14 wherein the vehicle or carrier is at least one of nanoparticles, microcapsules, microspheres, barriers, liposomes, lipid foams, solutions, osmotic pumps, fibers, filaments, gels and films.

16. The delivery vehicle as claimed in claim 12 or the composition as claimed in claim 14 wherein the vehicle or carrier comprises a polymer that is at least one polymer in the group of poloxamers, poly(orthoester)s, poly(vinyl alcohol)s, poly(anhydride)s, poly(methacrylate)s, poly(methacryladmide)s, anionic carbohydrate polymers, poly(hydroxybutyric acid)s, polyacetals, poly(1-lactide), poly(dl-lactide), poly(dl-lactide-co-glycolide)s, poly(1-lactide-co-glycolide)s, poly(e-caprolactone), polyglycolide, poly(p-dioxanone)s, poly(trimethylene carbonate), poly(alkylene diglycolate)s, poly(oxaester)s, poly(oxaamide)s and glyceride polymers.

17. The delivery vehicle as claimed in claim 12 or the composition as claimed in claim 13 wherein the vehicle or composition provides for single dose administration of the at least one histamine H4 receptor antagonist.

18. The delivery vehicle as claimed in claim 12 or the composition as claimed in claim 13 wherein the vehicle or composition provides for sustained release of the at least one histamine H4 receptor antagonist.

19. The delivery vehicle as claimed in claim 12 or the composition as claimed in claim 13 wherein the vehicle or composition provides for burst/sustained release of the at least one histamine H4 receptor antagonist.

20. The delivery vehicle as claimed in claim 12 or the composition as claimed in claim 13 comprising from about 0.001 milligram of the at least one histamine H4 receptor antagonist per kilogram of the body to about 200 milligrams of the at least one histamine H4 receptor antagonist per kilogram of the body.

21. The delivery vehicle or composition as claimed in claim 15 wherein the liposome is at least one of L-alpha-distearoyl phosphatidylcholine, phosphatidylcholine, dipalmitoylphosphatidylcholine, and distearoylphosphatidylcholine.

22. The delivery vehicle or composition as claimed in claim 15 wherein the solution comprises a crystalloid instillate, and said crystalloid instillate is at least one of phosphate buffered saline, saline and lactated Ringer's solution.

23. The delivery vehicle or composition as claimed in claim 15 wherein the solution comprises viscous instillate comprising a carrier, and said carrier comprises at least one of dextrans, cyclodextrans, hydrogels, carboxymethylcellulose, poly(saccharide)s, hyaluronic acids, crosslinked hyaluronic acids and chondroitin sulfates.

24. The histamine H4 receptor antagonist as claimed in claim 3 for use in the method according to claim 3, or the composition or delivery vehicle as claimed in claim 15 wherein the barrier is absorbable.

25. The composition or delivery vehicle as claimed in claim 24 wherein the absorbable barrier is at least one of hyaluronic acids, cellulosics derivatives, collagens, polyethylene glycols, pluronics, chitin, chitosans, dextrans, glucoses, carbohydrates, gelatins, glycosaminoglycans, polyacrylamides, polyvinyl pyrrolidones, polyvinyl alcohols, polymethyacrylics, aliginates, starches and polypeptides.

26. The delivery vehicle as claimed in claim 12 or the composition as claimed in claim 13 further comprising an additional therapeutic agent in an amount effective to provide the therapeutic effect intended by administration of the additional therapeutic agent.

27. The delivery vehicle or composition as claimed in claim 26 wherein the additional therapeutic agent is at least one of an anti-platelet, an anti-fibrotic, an antiinflammatory, an anti-proliferative and an agent that inhibits collagen synthesis.

28. A histamine H4 receptor antagonist for use in a method for the inhibition of post-operative adhesion formation in a body between tissue surfaces in a body cavity having been subjected to a surgical procedure, said method comprising administering an effective amount of at least one histamine H4 receptor antagonist systemically, wherein the at least one histamine H4 receptor antagonist is selected from the group consisting of:
benzoimidazol-2-yl pyrimidines and pyrazines;
pharmaceutically acceptable salts, hydrates, solvates and mixtures of said histamine H4 receptor antagonists.

29. The histamine H4 receptor antagonist as claimed in claim 28, wherein the administering is done before surgery.

30. The histamine H4 receptor antagonist as claimed in claim 28, wherein the administering is done after surgery.
